# EUROPEAN PATENT SPECIFICATION

(11) **EP 2 681 559 B1**
(45) Date of publication and mention of the grant of the patent: **05.12.2018**
(21) Application number: 12753030.1
(22) Date of filing: 05.03.2012
(51) Int. Cl.: G01N 33/555, C07K 14/00, C07F 9/10, C07K 17/06, G01N 33/53, G01N 33/531, G01N 33/536, C07K 14/045, G01N 33/68, A61K 39/385, A61K 31/683, A61K 38/16

(54) **PEPTIDE-LIPID CONSTRUCTS AND THEIR USE IN A FC-FUNCTION ASSAY**
PEPTID-LIPID-KONSTRUKTE UND IHRE VERWENDUNG IN EINEM FC-FUNKTIONSASSAY
CONSTRUCTIONS PEPTIDE-LIPIDE ET LEUR UTILISATION DANS UN TEST DE LA FONCTION FC

(30) Priority: 03.03.2011 NZ 59151411
(43) Date of publication of application: 08.01.2014
(73) Proprietor: Kode Biotech Limited, Auckland (NZ)
(72) Inventor: HENRY, Stephen Micheal, Auckland (NZ); KOMARRAJU, Venkata Sarvani, Auckland (NZ)
(74) Representative: Gowshall, Jonathan Vallance
(86) International application number: PCT/NZ2012/000029
(87) International publication number: WO 2012/118388

(56) References cited:
- WO-A1-2009/035347
- WO-A1-2009/048343
- WO-A1-2009/048343
- US-A1- 2003 119 039
- US-B1- 6 177 241
- T. GEORGAKOPOULOS ET AL: "An improved Fc function assay utilizing CMV antigen-coated red blood cells generated with synthetic function-spacer-lipid constructs", VOX SANGUINIS, vol. 102, no. 1, 12 July 2011 (2011-07-12) , pages 72-78, XP055125527,
- D HEATHCOTE ET AL: "IMMUNOHEMATOLOGY: Novel antibody screening cells, MUT+Mur kodecytes, created by attaching peptides onto red blood cells", TRANSFUSION, vol. 50, no. 3, 2010, pages 635-641, XP055125525,
- VRDOLJAK A ET AL: "A microassay for measurement of Fc function of human immunoglobulin preparations by using tetanus toxoid as antigen", BIOLOGICALS, vol. 32, no. 2, 2004, pages 78-83, XP004576876,
- "2.7.9 Test for Fc function of immunoglobulin" In: "European Pharmacopoeia 5.0", 2005, XP055141491, pages 202-203, * the whole document *
- J. NOVAK ET AL: JOURNAL OF GENERAL VIROLOGY, vol. 72, no. 6, 1991, pages 1409-1413, XP055141656,
- A SJÖHOLM: "Complement Components in Normal Serum and Plasma Quantitated by Electroimmimoassay", SCANDINAVIAN JOURNAL OF IMMUNOLOGY, vol. 4, no. 1, 1975, pages 25-30, XP055141486,
- HEATHCOTE D. ET AL.: 'Novel antibody screening cells, MUT+Mur kodecytes, created by attaching peptides onto red blood cells' TRANSFUSION vol. 50, no. 3, 2010, pages 635 - 641, XP055125525
- FRAME, T . ET AL.: 'Synthetic glycolipid modification of red blood cell membranes' TRANSFUSION. vol. 47, no. 5, 2007, pages 876 - 882, XP002593488
- GEORGAKOPOULOS T. ET AL.: 'An improved method for the determination of Fc function of Immunoglobulins' VOX SANGUINIS vol. 96, no. 2, 2009, pages 133 - 137, XP055125526
- GEORGAKOPOULOS, T. ET AL.: 'An improved Fc function assay utilizing CMV antigen- coated red blood cells generated with synthetic function-spacer-lipid constructs' VOX SANGUINIS vol. 102, no. 1, 2012, pages 72 - 78, XP055125527
- JIANG QIAN ET AL: "Simple synthesis of 1,3-cyclopentanedione derived probes for labeling sulfenic acid proteins", CHEMICAL COMMUNICATIONS, vol. 47, no. 32, 2011, page 9203, XP055327694,

## Description

### FIELD OF INVENTION

The invention relates to a method for assessing the Fc-function of immunoglobulin containing preparations. In particular, the invention relates to a method for assessing the Fc-function of intravenous immunoglobulin (IVIG) products.

### BACKGROUND ART

Intravenous immunoglobulin (IVIG) products are indicated as the treatment of choice in a number of immune-mediated disorders, including inflammatory diseases and autoimmune diseases (Riepert *et al* (2004)). Interactions with Fc-receptors have been described as an important mechanism of action of IVIG products. The Fc region of IgG mediates biological functions such as binding to Fc-receptors of macrophages for the promotion of phagocytosis, activation of the complement system and antibody-dependent cellular cytotoxicity (Anon (1997), Schroeder *et al* (2008), Virella (2005)). Evaluation of Fc-function is therefore, critical for assessing the biological activity of IVIG products and manufacturers are required to demonstrate that Fc-mediated functions are maintained in their IVIG products.

The test for the Fc-function of IVIG products (*European Pharmacopoeia,* Method 2.7.9) measures complement-mediated haemolysis of group O red blood cells (RBCs). The RBCs are tanninized and coated with Rubella antigen prior to contacting with samples of the IVIG product. Complement-mediated haemolysis is initiated by adding complement to the sample and haemolysis measured over time by monitoring absorbance (541 nm). The index of Fc-function (*I*_{Fc}) is derived from the time dependent absorbance curve. The test method (*European Pharmacopoeia* Method 2.7.9) employs a large number of preparative steps, a number of which may only be performed shortly before or at the time the test method is performed. As a consequence the test method is cumbersome and lengthy. The convenience and efficiency of the test method has been improved by using frozen RBCs and a microtitre plate format (Georgakopoulos *et al* (2009)). However, the test method remains problematic, particularly in respect of the preparation of the RBCs coated with Rubella antigen.

It is an object of this invention to provide an improved test method for the Fc-function of immunoglobulin containing preparations. It is an object of this invention to provide reduced intra- and inter-laboratory variation in assaying the Fc-function of immunoglobulin containing preparations. These objects are to be read disjunctively with the object to at least provide a useful choice.

### STATEMENT OF INVENTION

In a **first** aspect the invention provides a method of assaying Fc-function of a sample of an immunoglobulin containing preparation comprising the steps of:
- contacting the sample, in the presence of complement, with red blood cells (RBCs) modified to incorporate a construct of the structure F-S-L; and then
- monitoring the rate of haemolysis,
where F is a target antigen for a complement activating immunoglobulin, S is a spacer covalently linking F to L and selected to provide a construct (F-S-L) dispersible in water, and L is a lipid.

Preferably, where the complement is added to the sample following the step of contacting the sample with the RBCs the concentration of complement is in the range 18 to 28 CH_{50/}ml. More preferably, where the complement is added to the sample following the step of contacting the sample with the RBCs the concentration of complement is 23(±5%) CH₅₀/ml.

Preferably, the concentration of immunoglobulins in the sample is in the range 40 to 60 mg/ml. More preferably, the concentration of immunoglobulins in the sample is 50(±5%) mg/ml.

Preferably, the immunoglobulin containing preparation is serum. More preferably, the immunoglobulin containing preparation is an intravenous immunoglobulin (IVIG) product.

Preferably, the target antigen is an antigen of cytomegalovirus (CMV). More preferably, the target antigen is:
ThrProThrProValAsnProSerThrAlaProAlaProAlaProThrProThrPheAla
of the biosequence designated SEQ ID NO:1.

Preferably, the construct is of the structure: 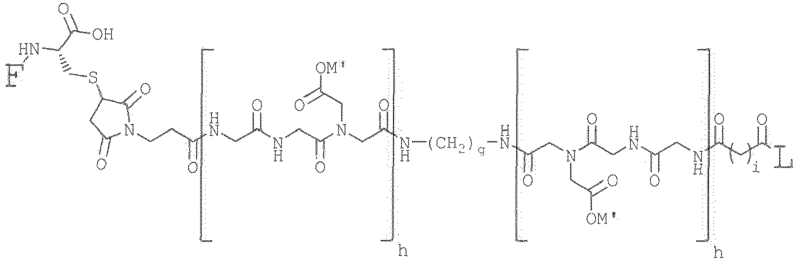 where g is the integer 1, 2 or 3, h is the integer 1, 2, 3 or 4, i is the integer 3, 4 or 5, and M' is a monovalent cation or substituent. An example of a monovalent cation is H⁺. An example of a monovalent substituent is methyl (CH₃). More preferably, the construct is of the structure;

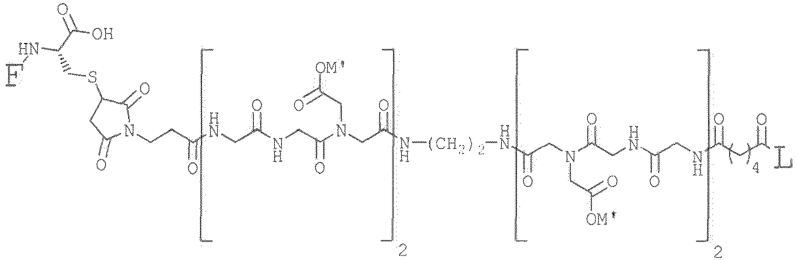

Preferably, the spacer (S) is covalently linked to the target antigen (F) via a cysteine residue at the carboxy (C) terminus of the target antigen.

Preferably, L is a diacyl- or dialkyl-glycerophospholipid. More preferably, L is a diacylglycerophospholipid. Preferably, the spacer (S) is covalently linked to the lipid (L) via an amide bond formed between the terminal carboxyl group and a primary amine. Preferably, L is a phosphatidylethanolamine. More preferably, L is a phosphatidylethanolamine of the structure: 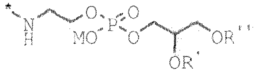 where M is a monovalent cation and R' and R" are independently selected from the group consisting of: oleoyl and stearyl. Typically, M is H. Most preferably, L is dioleoylphosphatidylethanolamine (DOPE).

In an embodiment of the first aspect of the invention the construct (F-S-L) is of the structure designated DOPE-Ad-CMG(2)-hCMV2

In a **second** aspect the disclosure provides constructs of the structure: 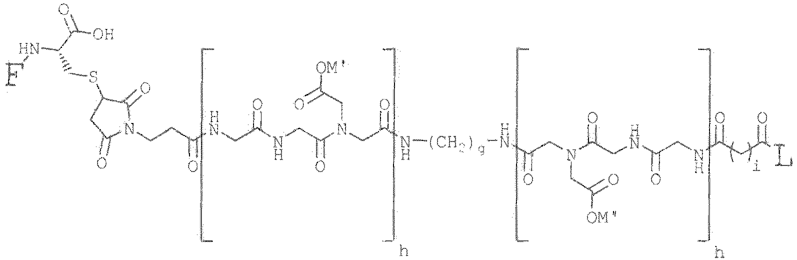 for use in a method of assaying the Fc-function of a sample of an immunoglobulin containing preparation where F is a peptide of the biosequence designated SEQ ID NO:1, L is phosphatidylethanolamine, g is the integer 1, 2 or 3, h is the integer 1, 2, 3 or 4, i is the integer 3, 4 or 5, and M' is a monovalent cation or substituent.

Preferably, g is 2, h is 2, i is 4, and M' is H.

In an embodiment of the second aspect of the disclosure the construct is of the structure: 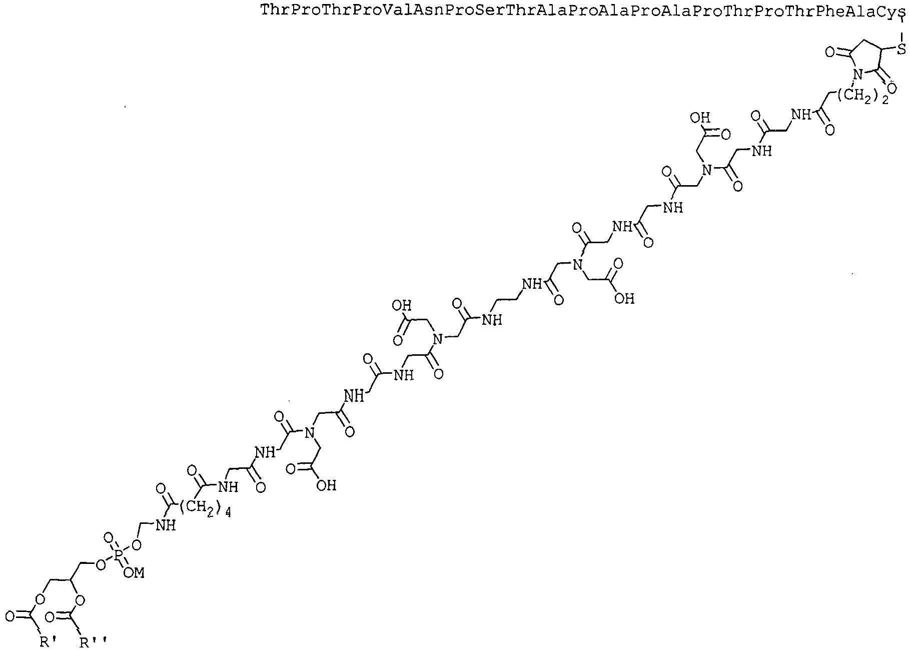 where R' and R" are both oleoyl, i.e. L is DOPE, and designated DOPE-Ad-CMG(2)-hCMV2.

In the description and claims of this specification the following acronyms, terms and phrases have the meaning provided: "Dispersible in water" means a stable, single phase system is formed in water at a concentration of at least 10 mg/ml in the absence of detergents or organic solvents.

"Complement activating immunoglobulin" means an immunoglobulin capable of activating the complement system.

"In the presence of complement" means the complement is either present in the sample prior to the step of contacting the sample with the RBCs, or added to the sample following the step of contacting the sample with the RBCs.

"Kodecytes" means red blood cells (RBCs) modified to incorporate one or more constructs of the structure F-S-L where F is a functional moiety, S is a spacer and L is a lipid.

"Modified to incorporate" means modified by incorporation of an exogenously preprared, preformed construct.

"Serum" means the amber-coloured, protein-rich liquid which separates out when blood coagulates.

"( )^{x}", "( )ₓ", "[ ]ₓ" and "[ ]^{x}" mean the group contained in the parentheses is repeated a number (x) of times. By way of illustration: 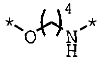 means the methylene group (-CH₂-) is repeated 4 times and the structure represented is equivalent to:

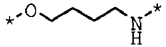

It will be recognised that the phosphate moiety of a phospholipid is often protonated, but the proton (H⁺) may be replaced by another monovalent cation such as Na⁺, K⁺, NH₄⁺ or triethylamine ([NH(CH₂CH₃)₃]⁺). Similarly, the primary and secondary amino functions of a construct may also be protonated. The constructs described in this specification may therefore be prepared as a number of salts, including pharmaceutically acceptable salts.

It should be noted that an asterix (*) is used to represent a point of covalent attachment to another moiety and does not indicate an atom.

In the description and claims of this specification the amino acids of peptides are identified in accordance with Annex C, Appendix 2 of the PCT Administrative Instructions (as in force from January 1, 2010) and in accordance with the convention:
["amino (N) terminus"] XaaXaaXaa......XaaXaaXaa ["carboxy (C) terminus"]

Where concentrations or ratios of reagents are specified the concentration or ratio specified is the initial concentration or ratio of the reagents. Where values are expressed to one or more decimal places standard rounding applies. For example, 1.7 encompasses the range 1.650 recurring to 7.499 recurring.

The terms "first", "second", "third", etc. used with reference to elements, features or integers of the subject matter defined in the Statement of Invention and Claims, or when used with reference to alternative embodiments of the invention are not intended to imply an order of preference.

The invention will now be described with reference to embodiments or examples and the figures of the accompanying drawings pages.

### BRIEF DESCRIPTION OF DRAWINGS

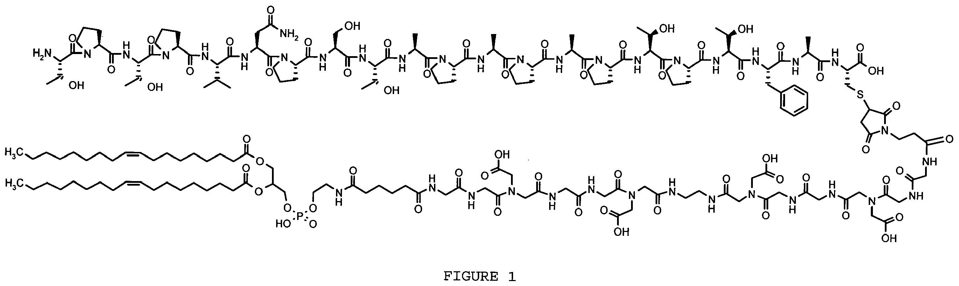

**Figure 1****.** Structure of the construct designated DOPE-Ad-CMG(2)-hCMV2 where M' and M'' are both H.
**Figure 2**. Haemolysis curves of kodecytes (modified at a concentration of 0.8 mg/ml of the construct designated DOPE-Ad-CMG(2)-hCMV2) following incubation with buffer (solid squares), IVIG (open triangles) or EPBRP standard (shaded circles) and the addition of 153 CH_{50/}ml of GPC. The samples were analysed in duplicate and the mean haemolysis curve for each sample is shown.

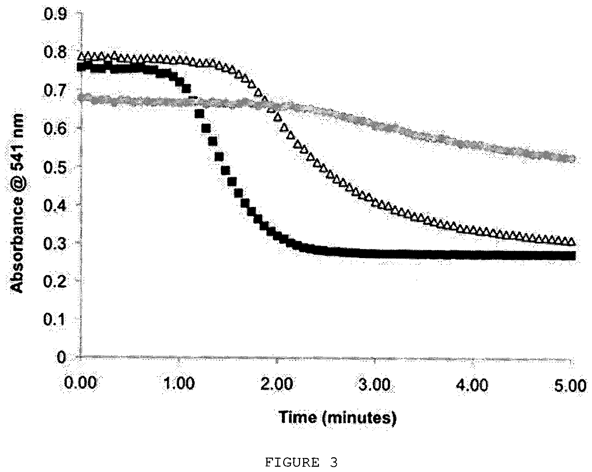

**Figure 3****.** The effect of different concentrations of complement on the rate of haemolysis of kodecytes (modified at a concentration of 0.8 mg/ml of the construct designated DOPE-Ad-CMG(2)-hCMV2) following incubation with EPBRP standard. Complement concentrations of 46 (solid squares), 23 (open triangles) and 11.5 (shaded circles) CH₅₀/ml were added to the samples in duplicate. The mean haemolysis curve for each concentration of complement is shown.

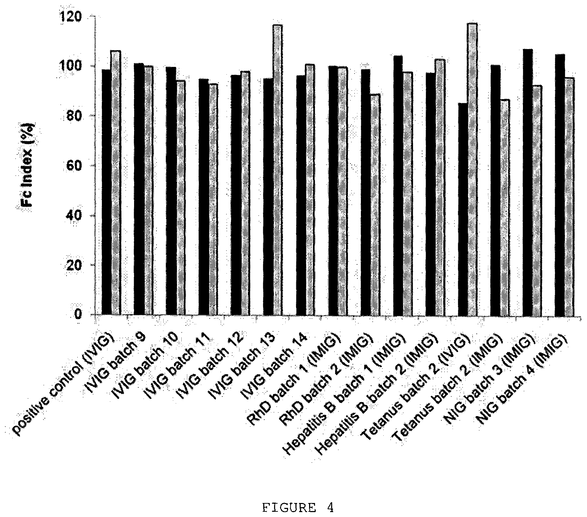

**Figure 4****.** A comparison between the Fc indices (*I_{Fc}*) of various samples of immunoglobulin containing preparations determined using kodecytes (solid squares) or the modified EP method according to Georgakopoulos *et al* (2009)(shaded squares).

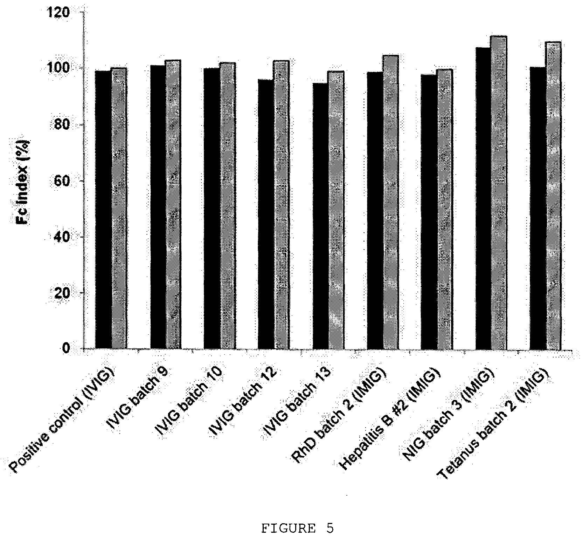

**Figure 5****.** Inter-batch variation of kodecytes in the method for assaying Fc-function. Samples of immunoglobulin containing preparations were incubated with batch #1 (solid squares) and batch #2 (shaded squares).

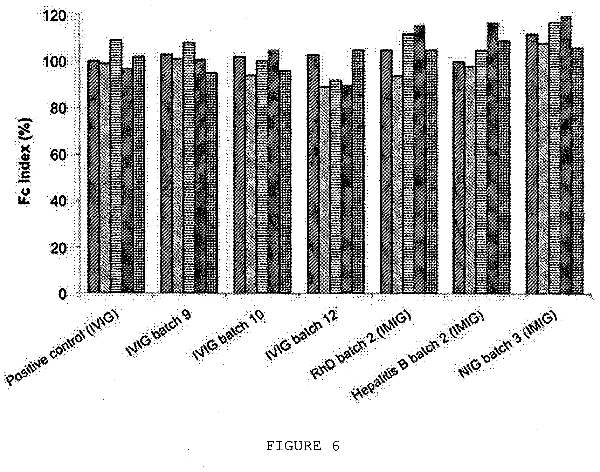

**Figure 6****.** Stability of kodecytes determined by comparing the Fc indices (*I_{Fc}*) obtained for samples of immunoglobulin containing preparations using kodecytes stored for 1 (dark shaded squares), 4 (diagonal hash squares), 6 (horizontal hash squares), 8 (light shaded squares) and 10 (cross hash squares) weeks at 2 to 8 °C.

### DETAILED DESCRIPTION

The invention resides in part in the selection of a target antigen for a complement activating immunoglobulin and its incorporation into a water dispersible construct that will spontaneously incorporate into the membranes of RBCs. Human cytomegalovirus (hCMV, human herpes virus 5), the beta herpes virus, is prevalent in human populations. Detection of the virus is part of the standard screening for blood donation. hCMV negative blood is particularly important for infants, organ transplantations and immunocompromised patients (Tutschka (1988)). As discussed in the specification accompanying international application no. PCT/NZ2008/000239 (publication no. WO 2009/035347) the use of kodecytes creates an opportunity to employ established blood typing platforms to detect a range of peptide antigen-antibody interactions with the capital costs associated with establishing a new diagnostic assay being avoided. The use of constructs (F-S-L) comprising the spacer (S) described in the specification accompanying international application no. PCT/NZ2008/000266 (publ. no WO 2009/048343) are advantageously used in the method of the present invention due to the absence (or at least negligible incidence) in the population of individuals expressing antibodies to the spacer (S). By contrast individuals expressing antibodies to polyethyleneglycol (PEG), for example, are known in the population.

In the context of assaying the Fc-funtion of immunoglobulin containing preparations, use of the method of the invention has greatly simplified and reduced the time of the assay (*cf. European Pharmacopoeia,* Method 2.7.9) by eliminating the need to freeze and thaw RBCs, treat with tannic acid and incubate with Rubella antigen. The insertion of the constructs (F-S-L) into the membrane to provide kodecytes is a simple incubation procedure requiring only two hours to perform. Furthermore, the kodecytes can be stored at 2 to 8°C for up to 10 weeks without loss of function, thus providing a readily available source for assaying Fc-function in samples of immunoglobulin containing preparations. In contrast, thawed RBCs, treated with tannic acid and derivatised with Rubella antigen are stable only for two days at 2 to 8°C. A further advantage of the invention is the ability to control the amount of antigen inserted into a cell membrane providing for improved batch-to-batch and laboratory-to-laboratory reproducibility in the preparation of kodecytes for use in the assay.

### EXAMPLES

### Materials

The construct designated DOPE-Ad-CMG(2)-hCMV2 was prepared according to the method described in the specification accompanying international application no. PCT/NZ2008/000266 (publ. no WO 2009/048343) employing the Cys terminated peptide ThrProThrProValAsnProSerThrAlaProAlaProAlaProThrProThrPheAlaCys. For the preparation of RBCs modified to incorporate the construct designated DOPE-Ad-CMG(2)-hCMV2 (kodecytes) the construct was dispersed in a cell preservative solution (CELPRESOL™, CSL Limited, Melbourne, Australia). RBCs of blood group O were obtained from the Australian Red Cross Blood Service (ARCBS). A European Pharmacopoeia Biological Reference Preparation (EPBRP) of human immunoglobulin (batch no. 3) was obtained from Council of Europe (Strasbourg, France). Guinea pig complement (GPC) was supplied by Harlan (Bicester, England). Purified and concentrated Rubella virus antigen of no less than 256 haemagglutination units was obtained from Aalto Bio Reagents (Dublin, Ireland). All samples of immunoglobulin containing preparations tested for Fc-function were obtained from CSL Biotherapies (Broadmeadows, Australia). The samples of immunoglobulin containing preparations tested included hyperimmune tetanus formulated at 6% (w/v) protein, pH 4.25 (IVIG) and hyperimmune hepatitis B and tetanus, RhD for intramuscular administration (IMIG), and normal immunoglobulin (NIG) formulated at 16% (w/v) protein, pH 6.8. Bovine serum albumin (BSA) fraction V was obtained from Calbiochem (La Jolla, CA, USA), tannic acid was obtained from Sigma (St Louis, MO, USA), pepsin was obtained from Roche Applied Science (NSW, Australia), phosphate buffered saline (PBS) was obtained from Pierce (Rockford, IL, USA), barbitone sodium was obtained from Merck (Darmstadt, Germany) and CELPRESOL™ was obtained from CSL Biotherapies (Parkville, Australia). Tris-glycine 4-20% polyacrylamide gradient gel, tris-glycine running buffer, tris-glycine non-reducing sample buffer and SeeBlue standard were obtained from Invitrogen (Mulgrave, Australia). Coomassie blue R-250 was obtained from Merck (Darmstadt, Germany), methanol (AR) and acetic acid (glacial) was obtained from APS Chemicals (Cheltenham, Australia). All solutions were prepared with pyrogen free water (PFW).

### Preparation of kodecytes

Packed O group RBCs were washed in saline then CELPRESOL™ and resuspended in CELPRESOL™. The Packed Cell Volume (PCV) of the suspension was adjusted to 50% ± 2.5% v/v with the addition of CELPRESOL™. A quantity of stock solution of the construct was added to the suspension and incubated at 37 °C for 2 hours with constant gentle agitation. Following incubation the kodecytes were washed with CELPRESOL™ and the PCV of suspension determined. The PCV of the suspension was adjusted 80% ± 2.5% v/v with the addition of CELPRESOL™ and the kodecytes stored at 2 to 8 °C.

### Fc-function assay using kodecytes

The kodecytes were spun at 3400 x g for 5 min at 4 °C. The CELPRESOL™ was removed and the kodecytes resuspended in albumin barbitone buffer (142 mM sodium chloride, 5 mM barbitone sodium, 0.15 mM calcium chloride, 0.5 mM magnesium chloride, 0.6 mM sodium azide and 0.15% w/v bovine serum albumin in PFW, pH 7.3) such that the absorbance of the suspension when diluted 1:10 was 1.0 ± 0.1 at 541 nm. The human immunoglobulin EPBRP standard and immunoglobulin containing preparations were diluted to 40, 50 or 60 mg/ml in albumin barbitone buffer solution and if necessary adjusted to pH 7 by addition of 0.5 M sodium hydroxide. Diluted samples (900 µl) were transferred to Eppendorf tubes. A negative control was also prepared by adding 900 µl of albumin barbitone buffer to an Eppendorf tube. Kodecytes (100 µl) were added to each Eppendorf tube and mixed. Samples were incubated for 1 hour before centrifugation at 2600 g for 1 minute. Supernatants were discarded and the kodecytes washed three times with 1 ml of albumin barbitone buffer. The kodecytes were resuspended in a final volume of 700 µl albumin barbitone buffer. Haemolysis was monitored in a microtitre plate format. A 240 µl aliquot of each sample of kodecytes was transferred in duplicate to wells of a microtitre plate and pre-warmed in a plate reader (SpectaMax 190, Molecular Devices, CA, USA) at 37 °C. A 60 µl aliquot of GPC diluted in albumin-barbitone buffer was pre-warmed for 30 seconds at 37 °C and added immediately to each sample well. The optimal concentration of GPC required for generating haemolyses curves that progressed at an appropriate rate was determined. The plate was returned to the plate reader and absorbance was monitored at 541 nm every 4 seconds for 12 minutes. The observed haemolysis curves were examined by electronic derivative analysis (Sigma Plot, Version 10.0, Systat Software, 2006, Chicago, IL) involving determination of the maximal rate of change of absorbance achieved at the inflection point (Georgakopoulos *et al* (2009)). The index of Fc-function was expressed as a percentage of the reference standard.

### Fc-function assay using modified European Pharmacopoeia method

Determination of Fc-function of samples of immunoglobulin containing preparations by a modified EP method was performed as previously described (Georgakopoulos *et al* (2009)).

### Pepsin digestion and analysis of an immunoglobulin containing preparation

Pepsin was added at 1.8 mg/ml to a sample of an IVIG product which was then incubated at 37 °C for 19 hours. The sample was adjusted to pH 7.4 with 0.5 M sodium hydroxide to inactivate the enzyme. Any precipitate was removed by centrifugation at 1000 g for 15 minutes. The protein concentration of untreated and pepsin-treated sample was determined by use of a biuret reagent. Untreated and pepsin-treated samples were loaded at 1 mg/ml on a 4 to 20% tris-glycine gradient gel under non-reducing conditions. When electrophoresis was complete the gel was stained with Coomassie blue for 30 minutes then destained and dried.

### Statistical analysis

Statistical analysis was performed using the Student's paired two tail t-test. A P value less than 0.05 was considered statistically significant.

### Results

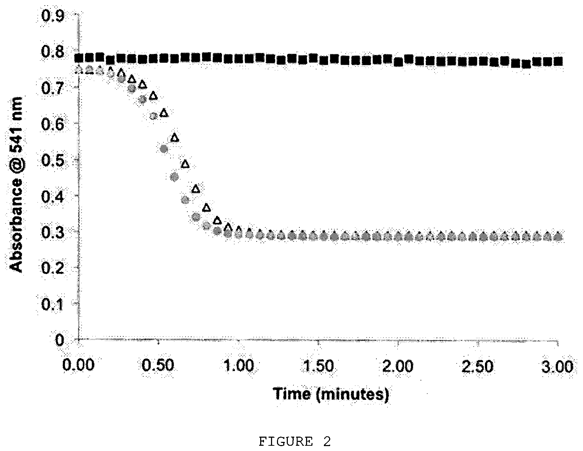

The twenty amino acid peptide (SEQ ID NO: 1) located between amino acids 595 to 614 on the pp150 structural protein of CMV has been reported to be immunogenic (Novak *et al* (1991), Ripalti *et al* (1994)). Figure 2 illustrates the haemolysis curves generated when the EPBRP standard and an intravenous immunoglobulin preparation (positive control) at 40 mg/ml were incubated for 1 hour with kodecytes prepared with a solution of the construct designated DOPE-Ad-CMG(2)-hCMV2 at a concentration of 0.8 mg/ml. Although, the reaction rate following the addition of 153 CH₅₀/ml complement was too rapid the results appeared promising as the haemolysis curves of IVIG and the EPBRP standard were comparable. At a complement concentration of 23 CH_{50/}ml, haemolysis was complete within 3 min. Immunoglobulin was used at a concentration of 50 mg/ml in the assay to ensure highest possible availability of anti-CMV for binding to the kodecytes. With appropriate Fc-function assay parameters defined for using kodecytes, studies were undertaken to validate the assay by determining the inter- and intra-assay variation. For validation of a bioanalytical method it is recommended that the precision around the mean value should not exceed 15% of the coefficient of variation (CV) (Anon (not known)). The inter-assay precision was determined by testing 16 different immunoglobulin samples in duplicate in six separate assays over a period of two weeks. The Fc indices for all products tested were consistent with the expected values and the CV was between 5 and 12% which satisfied the predefined acceptance criteria of less than 15%. The intra-assay precision was determined by testing one IVIG sample eight times within the same assay. The Fc index of the IVIG sample reflected the expected value and the CV was found to be 6% which satisfied the acceptance criteria. A comparison of Fc-function determined by the EP method and using kodecytes was performed. The Fc-function of various immunoglobulin products was determined using the two methods. No significant difference was observed in the results by the two methods. Taken together, these results display that the use of kodecytes is a valid approach for determining Fc-function. To establish the inter-batch variation of kodecytes, the Fc-function of various immunoglobulin products was determined using two different batches of kodecytes. The CV for the products tested using different batches of kodecytes was between 1 and 7% which satisfied the acceptance criteria and demonstrated that different batches will generate similar results. The robustness of the kodecytes was established by comparing the Fc indices of various immunoglobulin preparations generated using kodecytes stored for 1, 4, 6, 8 and 10 weeks at 2 to 8 °C. The results revealed comparable Fc indices between each storage time and the CV ranged from 4 to 8% for each product tested. CMV kodecytes can therefore be stored up to 10 weeks at 2 to 8 °C without loss of function. To verify that the Fc-function assay with the kodecytes was specific for intact IgG, an IVIG sample was treated with pepsin overnight to generate F(ab)₂ and Fc fragments. To confirm fragmentation of IgG, the untreated and pepsin-treated IVIG samples were run on an SDS-PAGE gel (data not shown). The Fc function of undigested and pepsin-treated IVIG was evaluated using CMV kodecytes. The untreated IVIG sample revealed an Fc index of 99% whereas the pepsin-treated sample showed a significantly lower Fc index of 28%. The low level of complement-induced haemolysis observed with the pepsin-treated sample was most likely due to the presence of a low level of uncleaved IgG (as seen by SDS-PAGE electrophoresis). These results verified that the Fc-function assay is specific for intact IgG molecules.

### REFERENCES

Anon (1997) B cell receptors, immunoglobulin and Fc receptors Klein J, Horejsi V (eds): Immunology. London, Blackwell Science, 204-270.
Anon (2007) Test for Fc function of immunoglobulin European Pharmacopoeia (Monograph 2.7.9), Edition 6, Volume 1. Strasbourg, European Directorate for the Quality of Medicines & HealthCare.
Anon (not known) Guidance for Industry - bioanalytical method validation; http://www.fda.gov/downloads/Drugs/GuidanceComplianceRegulatoryInformatio n/Guidances/ucm070107.pdf
Beers, M.H. (ed) (2006) Merck Manual of Diagnosis and Therapy 18th Edition, Merck Research Laboratories.
Frame et al (2007) Synthetic glycolipid modification of red blood cell membranes Transfusion Vol 47, 876-882.
Galili et al (1984) A unique natural human IgG antibody with anti-α-Galactosyl specificity J. Exp. Med Vol 160, 1519-1531.
Georgakopoulos et al (2009a) An improved Fc function assay utilizing CMV antigen-coated red blood cells generated with synthetic function-spacer-lipid constructs Vox Sang Vol 96, 133-137.
Georgakopoulos et al (2009b) An improved method for the determination of Fc function of immunoglobulins Vox Sang 96, 133-137.
Georgakopoulos et al (2011) An improved method for the determination of Fc function of immunoglobulins Vox Sang, 1-7.
Gronski et al (1991) Measurement of Fc function on human immunoglobulin preparations Pharmeuropa 3, 287-292.
Gurevich et al (2006) Determination of Fc function with frozen red blood cells Biologicals 34, 221-222.
Harrison et al (2010) A synthetic globotriaosylceramide analogue inhibits HIV-1 infection in vitro by two mechanisms Glycoconj J Vol 27, 515-24.
Heathcote et al (2010) Novel antibody screening cells, MUT+Mur kodecytes, created by attaching peptides onto red blood cells Transfusion Vol 50, 635-641.
Henry (2009) Modification of red blood cells for laboratory quality control use Curr Opin Hematol Vol 16, 467-72.
Hooper, JA (1997): Production and properties of intravenous immunoglobulins Lee ML, Strand V (eds): Intravenous Immunoglobulins in Clinical Practice. New York, Marcel Dekker, 37-55.
Jorquera, JI (2009) Flebogamma 5% DIF development: rationale for a new option in intravenous immunoglobulin therapy Clin Exp Immunol 157, 17-21.
Macher and Galili (2008) The Gala1,3Galb1,4GlcNAc-R (a-Gal) epitope: A carbohydrate of unique evolution and clinical relevance Biochim. Biophys. Acta Vol 1780, 75-88.
Novak et al (1991) Mapping of serologically relevant regions of human cytomegalovirus phosphoprotein ppl50 using synthetic peptides J Gen Virol Vol 72, 1409-13.
Riepert et al (2004) Evaluating the Fc-Function of Intravenous Immunoglobulin Products by Flow Cytometry J Allergy Clin Immunol S214, 751
Ripalti et al (1994) Construction of polyepitope fusion antigens of human cytomegalovirus ppUL32: reactivity with human antibodies J Clin Microbiol Vol 32, 358-63.
Schroeder et al (2008) Immunoglobulins: structure and function Paul WE (ed.): Fundamental Immunology. Philadelphia, Lippincott Williams & Wilkins, 125-150.
Virella, G (2005) Biosynthesis, metabolism and biological properties of immunoglobulins Virella G (ed.): Medical Immunology New York, Marcel Dekker, 109-121.
Vlug et al (1994) Nephelometric measurements of human IgG subclasses and their reference ranges Ann Biol Clin 52, 561-567.
von Gunten et al (2009) Intravenous immunoglobulin contains a broad repertoire of anticarbohydrate antibodies that is not restricted to the IgG2 subclass J Allergy Clin Immunol Vol 123, 1268-76.
European Pharmacopoeia (Monograph 2.7.9) Test for Fc function of immunoglobulin in, Edition 6, Volume 1. Strasbourg, European Directorate for the Quality of Medicines & HealthCare, 2007

## Claims

1. A **method** of assaying Fc-function of a sample of an immunoglobulin containing preparation, preferably serum, comprising the steps of:
• contacting the sample, in the presence of complement, with red blood cells modified to incorporate a construct of the structure F-S-L; and then
• monitoring the rate of haemolysis,
where F is a target antigen for a complement activating immunoglobulin, S is a spacer covalently linking F to L and selected to provide a construct dispersible in water, and L is a lipid.

2. The method of claim 1 where the immunoglobulin containing preparation is an intravenous immunoglobulin product.

3. The method of claim 2 where the target antigen is an antigen of cytomegalovirus.

4. The method of claim 3 where the target antigen is the peptide of the biosequence designated SEQ ID NO: 1.

5. The method of claim 1 where the construct is of the structure: 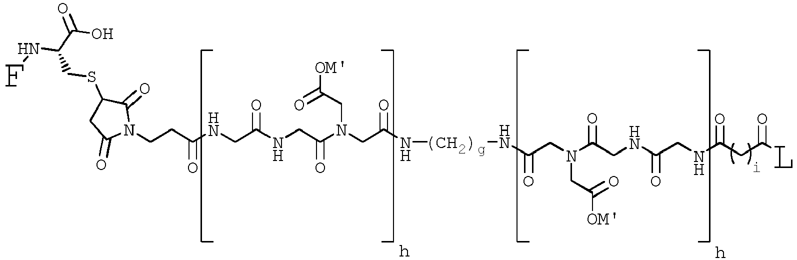 where g is the integer 1, 2 or 3, h is the integer 1, 2, 3 or 4, i is the integer 3, 4 or 5, and M' is a monovalent cation or substituent.

6. The method of claim 5 where the construct is of the structure:

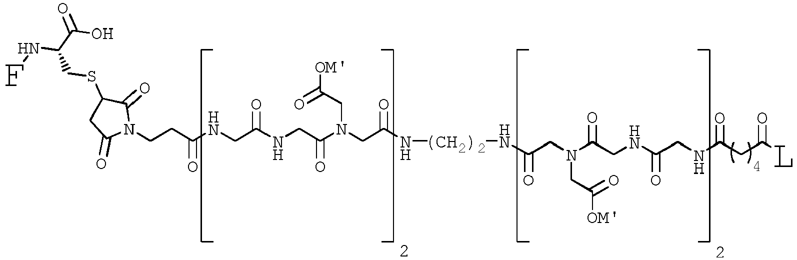

7. The method of claim 1 where L is a diacyl- or dialkyl-glycerophospholipid.

8. The method of claim 7 where L is a diacylglycerophospholipid.

9. The method of claim 8 where L is a phosphatidylethanolamine.

10. The method of claim 9 where L is a phosphatidylethanolamine of the structure: 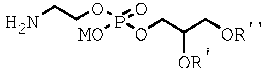 where M is a monovalent cation and R' and R" are independently selected from the group consisting of oleoyl and stearyl.

11. The method of claim 10 where L is dioleoylphosphatidylethanolamine (DOPE) .

12. The method of any one of claims 1 to 11 where the complement is present at a concentration in the range 18 to 28 CH₅₀/ml.

13. The method of claim 12 where the complement is present at a concentration of 23(±5%) CH_{50/}ml.

14. The method of any one of claims 1 to 13 where the concentration of immunoglobulins in the sample is in the range 40 to 60 mg/ml.

15. The method of claim 14 where the concentration of immunoglobulins in the sample is 50(±5%) mg/ml.

## Patentansprüche

1. Verfahren zum Prüfen von Fc-Funktionen einer Probe einer immunglobulinhaltigen Zubereitung, vorzugsweise Serum, das die folgenden Schritte umfasst:
• Inkontaktbringen der Probe mit roten Blutkörperchen, die so modifiziert sind, dass sie ein Konstrukt der Struktur F-S-L einbeziehen, in Gegenwart von Komplement; und dann
• Überwachen der Hämolysegeschwindigkeit,
wobei F ein Zielantigen für ein komplementaktivierendes Immunglobulin ist, S ein Spacer ist, der F kovalent mit L verbindet und so ausgewählt ist, dass er ein in Wasser dispergierbares Konstrukt bereitstellt, und L ein Lipid ist.

2. Verfahren nach Anspruch 1, wobei die immunglobulinhaltige Zubereitung ein intravenöses Immunglobulinprodukt ist.

3. Verfahren nach Anspruch 2, wobei das Zielantigen ein Antigen des Cytomegalovirus ist.

4. Verfahren nach Anspruch 3, wobei das Zielantigen das Peptid der Biosequenz mit der Bezeichnung SEQ ID Nr. 1 ist.

5. Verfahren gemäß Anspruch 1, wobei das Konstrukt die folgende Struktur aufweist: 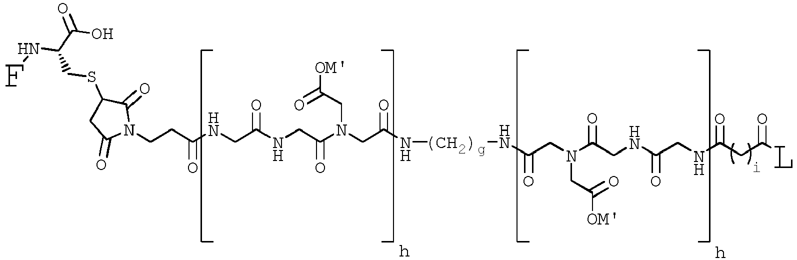 wobei g die ganze Zahl 1, 2 oder 3 ist, h die ganze Zahl 1, 2, 3 oder 4 ist, i die ganze Zahl 3, 4 oder 5 ist und M' ein monovalentes Kation oder ein Substituent ist.

6. Verfahren gemäß Anspruch 5, wobei das Konstrukt die folgende Struktur aufweist:

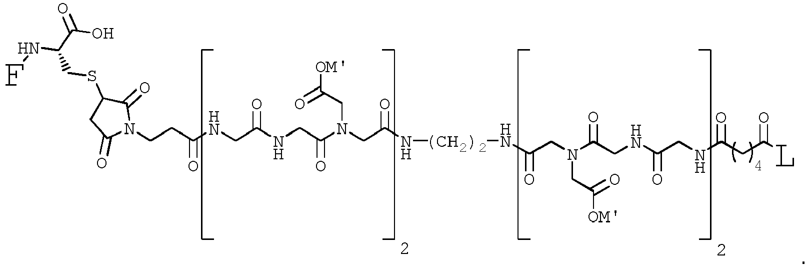

7. Verfahren nach Anspruch 1, wobei L ein Diacyl- oder Dialkylglycerophospholipid ist,

8. Verfahren nach Anspruch 7, wobei L ein Diacylglycerophospholipid ist.

9. Verfahren nach Anspruch 8, wobei L ein Phosphatidylethanolamin ist.

10. Verfahren nach Anspruch 9, wobei L ein Phosphatidylethanolamin mit der folgenden Struktur ist: 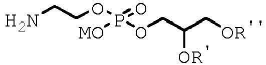 wobei M ein monovalentes Kation ist und R' und R'' unabhängig voneinander aus der Gruppe ausgewählt sind, die aus Oleoyl und Stearyl besteht.

11. Verfahren nach Anspruch 10, wobei L Dioleoylphosphatidylethanolamin (DOPE) ist.

12. Verfahren nach einem der Ansprüche 1 bis 11, wobei das Komplement in einer Konzentration im Bereich von 18 bis 28 CH₅₀/ml vorliegt.

13. Verfahren nach Anspruch 12, wobei das Komplement in einer Konzentration von 23(±5%) CH₅₀/ml vorliegt.

14. Verfahren nach einem der Ansprüche 1 bis 13, wobei die Konzentration des Immunglobulins in der Probe im Bereich von 40 bis 60 mg/ml liegt.

15. Verfahren nach Anspruch 14, wobei die Konzentration des Immunglobulins in der Probe 50(±5 %) mg/ml beträgt.

## Revendications

1. Procédé d'essai de la fonction Fc d'un échantillon d'une préparation, de préférence d'un sérum, contenant des immunoglobulines, comprenant les étapes consistant à :
• mettre en contact l'échantillon, en présence de complément, avec des globules rouges modifiés pour incorporer un produit de construction de la structure F-S-L ; puis
• surveiller la vitesse d'hémolyse,
où F est un antigène cible pour une immunoglobuline activant le complément, S est un espaceur liant de façon covalente F à L et choisi pour fournir un produit de construction dispersible dans l'eau, et L est un lipide.

2. Procédé selon la revendication 1, dans lequel la préparation contenant des immunoglobulines est un produit d'immunoglobulines intraveineuses.

3. Procédé selon la revendication 2, dans lequel l'antigène cible est un antigène de cytomégalovirus.

4. Procédé selon la revendication 3, dans lequel l'antigène cible est le peptide de la bioséquence désignée SEQ ID NO: 1.

5. Procédé selon la revendication 1, dans lequel le produit de construction est de la structure : 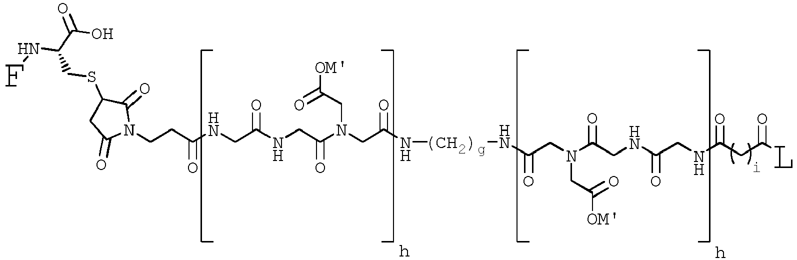 où g est l'entier 1, 2 ou 3, h est l'entier 1, 2, 3 ou 4, i est l'entier 3, 4 ou 5, et M' est un cation ou substituant monovalent.

6. Procédé selon la revendication 5, dans lequel le produit de construction est de la structure :

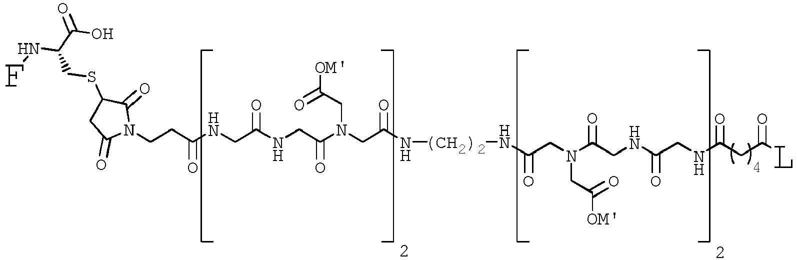

7. Procédé selon la revendication 1, dans lequel L est un diacyl- ou dialkyl- glycérophospholipide.

8. Procédé selon la revendication 7, dans lequel L est un diacylglycérophospholipide.

9. Procédé selon la revendication 8, dans lequel L est une phosphatidyléthanolamine.

10. Procédé selon la revendication 9, dans lequel L est une phosphatidyléthanolamine de la structure : 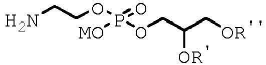 où M est un cation monovalent et R' et R" sont indépendamment choisis dans le groupe consistant en oléoyle et stéaryle.

11. Procédé selon la revendication 10, dans lequel L est la dioléoylphosphatidyléthanolamine (DOPE).

12. Procédé selon l'une quelconque des revendications 1 à 11, dans lequel le complément est présent à une concentration dans la plage 18 à 28 CH₅₀/ml.

13. Procédé selon la revendication 12, dans lequel le complément est présent à une concentration de 23(±5 %) CH₅₀/ml.

14. Procédé selon l'une quelconque des revendications 1 à 13, dans lequel la concentration d'immunoglobulines dans l'échantillon est dans la plage 40 à 60 mg/ml.

15. Procédé selon la revendication 14, dans lequel la concentration d'immunoglobulines dans l'échantillon est de 50(±5 %) mg/ml.
